# EUROPEAN PATENT SPECIFICATION

(11) **EP 0 695 157 B1**
(45) Date of publication and mention of the grant of the patent: **25.03.1998**
(21) Application number: 93913688.3
(22) Date of filing: 23.04.1993
(51) Int. Cl.: A61F 13/00, A61F 15/00

(54) **DISPOSABLE WATERPROOF COVERING**
WEGWERFBARER WASSERUNDURCHLÄSSIGER SCHUTZARTIKEL
PROTECTION IMPERMEABLE A L'EAU JETABLE

(43) Date of publication of application: 07.02.1996
(73) Proprietor: ELIASSON, Torbjörn, S-165 77 Hässelby (SE); JOHANSSON, Ove, S-722 13 Västeras (SE)
(72) Inventor: JOHANSSON, Ove, S-722 13 Västèras (SE)
(74) Representative: Bjellman, Lennart Olov Henrik
(86) International application number: SE9300358
(87) International publication number: WO9424971

(56) References cited:
- US-A- 4 727 864
- US-A- 4 911 151

## Description

The present invention relates to a disposable, protective covering which is passed over a body limb to protect it against moist and wet upon bathing and showering, particularly when this is called for due to a wound on an arm or a leg, which accordingly is dressed with a bandage or a plaster support. More particularly, the invention relates to a sleeve made from a material that is impermeable to water and has means for a leak proof closure of the sleeve around the extremity, above the wound or dressing, as well as a method for producing such sleeve and the utilization of a composite plastic for manufacturing the sleeve.

The problem in designing an operative protection includes above all the provision of a leak proof closure of the sleeve in contact with the body limb.

Examples on efforts to provide this leak proof closure can be found in several documents, and the US patent 4 911 151 thus discloses a sleeve made from polyethylene with a closure strap, preferably made of polyurethane, attached near the open end of the sleeve. The closure strap is highly elastic and has a width of 1/2 to 3/4 of an inch, i.e. about 12-18 mm. One end of the strap is permanently attached to the sleeve, while the other end has a pressure sensitive adhesive and is attached to the outside of the sleeve after being wound and tightened around the sleeve and around the body limb to be protected.

An arrangement of this type, however, presents several disadvantages. E.g., the high elasticity of the closure strap would involve a risk of the strap being stretched too tight around the body limb and might thus disturb the flow of blood. A further disadvantage is that the closure strap, due to several interacting causes, in most cases cannot prevent water to find its way through to the wounded limb. One of these causes is the positioning of the closure strap below the opening of the sleeve, which causes the sleeve to form a pouch above the closure strap, leading to accumulation of water on the inside of the sleeve, above the closure. Another reason is that the strap cannot prevent, due to its small width and by not adhering to the outside of the sleeve along its full length, that adjoining areas of the sleeve inside, i.e. within the folds formed around the body part upon tightening of the closure strap, will be displaced relative one another when the extremity is bent under washing, whereby water from the formed pouch will successively penetrate further into the closure and finally also into the wounded body part. This negative effect is increased by the construction of the closure strap in combination with the chosen material for the sleeve. Without the incorporation of an additional ingredient, the polyethylene itself is not sufficiently ductile and smooth to form pleats without the creation of fine channels in the pleating. Furthermore, the unmixed polyethylene has a very low surface friction, which adds to the above said relative displacement between adjoining areas in the pleating. A careful selection and composition of materials for the protective sleeve has significative importance for achieving the desired leak proof closure, which will be explained more in detail below in conjunction with the disclosure of the inventive protective sleeve.

In US-A-4 727 864 reference is also made to the above said feature of "synthetic materials" which upon folding "form fine channels through which water can penetrate", and in this document a protective sleeve with a closure strap is disclosed, which is attached transversely to the longitudinal axis of the sleeve. Along its entire length, the strip is provided with an adhesive layer, and upon sealing the sleeve, the strap is intended to adhere to the sleeve outside with its lower half and with its upper half to adhere to the skin of the extremity, so that the opening edge of the sleeve is covered by the strap, thereby preventing water to penetrate into the channels, formed by the pleating. On the inside of the opening edge, this protective sleeve further comprises an adhesive strip for temporary securing of the sleeve, and in an alternative embodiment a sponge rubber band is attached to the adhesive side of the closure strap.

Provided that the application is done thoroughly, this arrangement should offer a satisfactory closure of the sleeve. However, the added sponge rubber band indicates that the adhesion of the closure strap is not fully reliable, considering in particular the adhesion to the skin, and thus additional closure means must be added to lock out the water. Accordingly, said construction is above all concentrated on preventing the water to reach the edge of the protective sleeve, and provides no means to obtain a leak proof contact between the sleeve and the skin of the wounded body part. In other words, it has not been within the scope of invention to provide a protective sleeve with optimum material characteristics, to cooperate with a satisfactory operating closure means for providing a water proof closure, as is the case with the present invention.

Beside the above cited documents, reference can also be made to the US patents 3 324 580 and 3 906 941, both of which describe protective sleeves with separate closure means, in first said case in the form of a wide strap, partly provided with an adhesive layer, and which is attached to the skin above the edge of the sleeve and is hanging over and surrounding the upper end of the sleeve in the form of a collar, and in the second case in the form of a stretchable strap, which is wrapped to overlap the upper edge of the sleeve and is secured by a specifically shaped closure tab and an elastic band, forming a loop.

However, both constructions suffer from the disadvantage that arise from the sole fact that the cover is formed by several parts, which complicates the handling in all stages starting from the manufacturer to the user, and further disadvantages related to the specific construction can be found in the respective case. As for 3 324 580, there is a clear risk for leakage to arise since the collar might operate as a lever and dislocate the closure strap, especially in a bathing situation and undoubtedly when applied to children, playing in the water. As for 3 906 941, a too great part in creating the desired effect is left over to the user, who has to perform this wrapping "tight enough to prevent water from entering...., but not so tight so as to reduce the circulation of blood to the leg of the user", to have anyone readily and without reservation assigning the construction any technical effect of its own. Furthermore, there is a hazard that the stretchable closure strap will slide off the spool shaped extremity, since the strap has no adhesive layer.

Finally reference can also be made to the US patents Nos. 3.741.203, 3.747.125 and 4.562.834, which describes comparatively complicated arrangements, and cannot be considered as embodiments of the same category of protection as the present invention, which even at the stage of the user is aimed at having a price, sufficiently low to make it a disposable article.

Considering the above-said, the object of the present invention is to provide a shower and bath protection, which besides providing a leak proof sealing with the body part by appropriate combination of the specific features of the incorporated materials and the constructive arrangement of the closure means, also complies with the demands for low manufacturing costs which are raised for a disposable product.

A further object of the present invention is to provide a shower and bath protection in which the incorporated materials are skin-and environmently friendly, and will not cause allergic reactions.

To meet with above said objects, the inventive shower and bath protection will be constituted according to the attached claims 1 6 and 7, wherein the characterizing features of the invention are apparent. These characteristics also clearly define the differences between the shower protection of the present application and the shower protection of our former Swedish patent application no. 90 01719-5 (= WO-A-9117733), which forms the preamble of independent claims 1 and 6.

Briefly, the shower and bath protection of the present invention includes a sleeve, manufactured by cutting to proper length a tube of thin plastic material and heat sealing the tube in one end, whereby a sleeve without side seams is formed. A closure strap is attached adjacent to the open end edge of the sleeve, the strap extending in the longitudinal direction of the sleeve and having preferably the same length. One side of the strap comprises an adhesive layer, covered by a protective foil that is removed when applying the sleeve, whereby in a first revolution the strap is wrapped around the outside of the protective sleeve and in a second revolution is wrapped partly over the skin of the wounded body part and partly overlapping itself.

The protective sleeve and closure strap comprises composite materials that advantageously cooperate to provide an operative water protection, which will be explained more in detail below in conjunction with an embodiment of the invention, and with reference made to the attached drawings, of which:
- Figure 1: in a perspective view shows the shower and bath protection according to the invention,
- Figure 2: is an elevational, diagrammetic view of the protective sleeve open end, which shows the attachment between the protective sleeve and the closure strap,
- Figure 3: shows, in a first step, the application of the closure strap,
- Figure 3a: shows the forming of a tab of excessive material in said first application step,
- Figure 4: shows the application of the closure strap in a second step, and
- Figure 5: shows a ventilating valve for ventilation of the protective sleeve when using it as a bath protection.

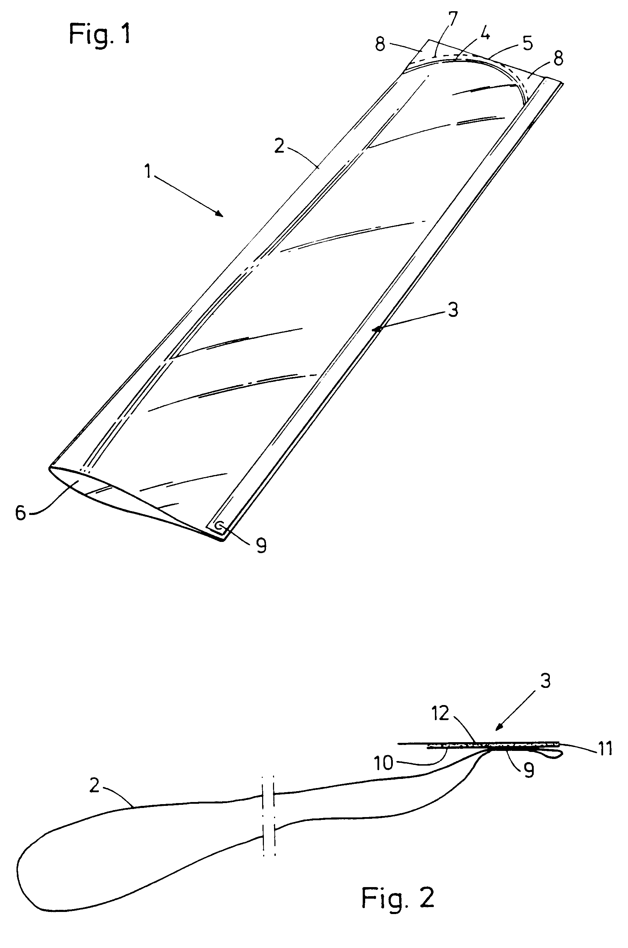

Figure 1 shows the shower and bath protection of the invention, generally referred to by the reference number 1. The protection 1 substantially comprises two parts, the sleeve 2 and the closure strap 3. The sleeve 2 is formed by heat sealing one end of a thin plastic tube along a sealing line 4, and cutting the tube to the desired length along the cutting line 5, i.e. along the cutting line that will produce the edge of the opening 6. Preferably, the closure strap 3, that is attached to the sleeve, can be cut in the same step, whereby the strap will have the same length as the sleeve 2. In conjunction with the sealing of the sleeve along the sealing line 4, a perforation 7 can optionally be made peripherally of the line 4, whereby the excessive material 8 in a successive step can be removed from the sleeve 2.

The closure strap 3, which accordingly has substantially the same length as the sleeve 2, is connected to the sleeve through an attachment 9, in the vicinity of the edge of the opening 6, as is apparent from Figure 1 and is shown diagrammatically and in larger scale in Figure 2. The strap 3 comprises a tape 10, which includes a synthetic carrier with an adhesive layer 11 and a protective foil 12. From Figure 2 is apparent, that the strap 3 is attached to the sleeve 2, preferably by a welded attachment 9, joining the synthetic carrier of the tape 10 with both layers of the longitudinally seamless and flattened sleeve 2. This can be made without affecting the operation of the protective foil by choosing, for the later, a material that is not damaged by the heat applied upon welding. In the present invention, the protective foil 12 is for this reason chosen form a silicone treated paper, such as the protective foil put to market by finnish Lohjan Paperi Oy under the trademark RELEASE ESP 48. This release paper is of environmently friendly quality and suitable as a protective foil for surgical tapes, and is provided in a width of 3 cm, suitable for this purpose.

The tape 10 of present invention includes a transparent carrier of polyethylene, coated along its entire length with an adhesive layer 11 such as polyacrylate, and is friendly to the skin.

Advantageously, a tape sold under the trade mark Scanpor by Norgesplaster AS, Norway, can be utilized. The tape 10 has a width of 2.5 cm, whereby the protected foil 12 is overlapping the tape by 0.5 cm along the length of the closure strap 3, which facilitates the removal of the protective foil.

As a basic material for the protective sleeve 2 of the present invention is selected a composition of polyethylene and EBA (Ethylene-Butyl-Acrylate), which has proved to be a quality that combined with the construction of the closure means provides an effective water protection. The ratio of mixture is in the range of 10-20 percent by weight of EBA, preferably 15-18 percent, and the material thickness is preferably in the range of 0.025-0.035 mm. The quality provided by this ratio of mixture comprises in comparison with the unmixed polyethylene a greater smoothness and softness, a less slippery surface, i.e. a higher surface friction, and even a greater stretchability before the rupture limit is reached. These features are of utmost value for the creation of a leak proof closure between the protective sleeve and the skin of the extremity to be protected.

Because of the better smoothness of the composite plastic in the protective sleeve 2, it has an improved ability than previously known sleeves, made solely from polyethylene, to form pleats around the wounded body part, with significantly less tendency to form channels in the folds. Furthermore, the higher surface friction decreases the risk of relative movement between contacting surface regions of the pleats, a problem which is additionally reduced by the closure strap comprising an adhesive layer along its entire length, and thus adheres to the sleeve outside, all around the body part. The high stretchability of the material also means, that even a very tautly applied protective sleeve will be able to provide a leak proof closure, since the material more easily will stretch over the elbow of a bended arm or over the knee of a bended leg, and will not immediately dislocate the sealing with the extremity upon movement.

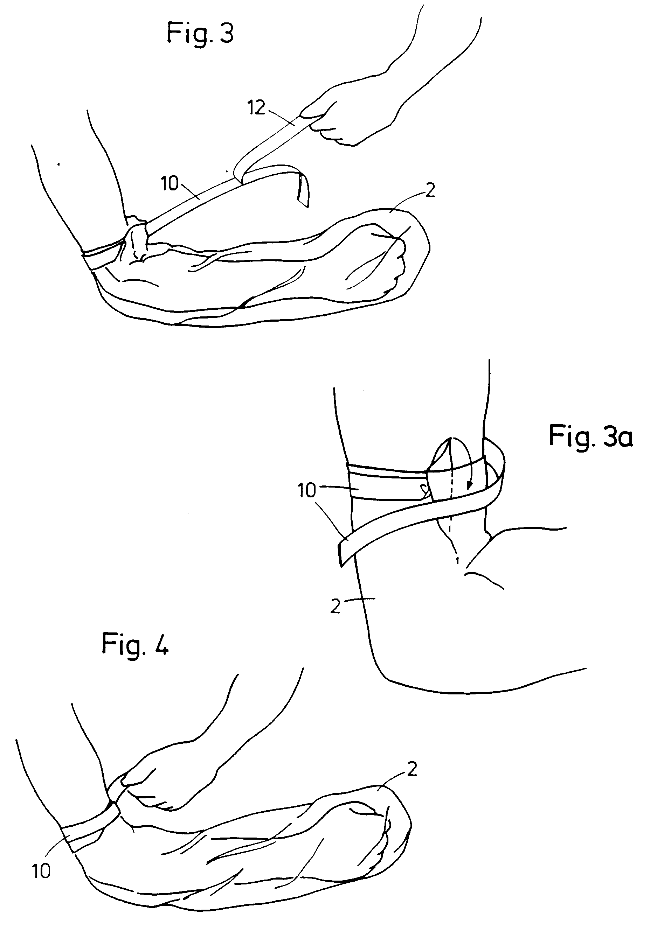

Figure 3 shows the way the protective sleeve 2, passed over en arm, in a first application step is brought to seal with the skin by removing the protective foil 12 and winding the closure strap 3 around the sleeve to secure, with its adhesive layer, the sleeve opening edge in a pleating with close contact to the skin. In that respect, its advantageous for obtaining the best result, to allow the excessive material to form one large, single fold close to the closure strap attachment point, which can be facilitated by winding the strap always in the same direction and to the right, as seen from the user. Upon forming the fold or pleat, a tab is formed (see Figure 3a) which is stretched and folded back over the sleeve edge and secured with the tape when finishing the first revolution, which should substantially be located inside or near the sleeve edge.

Figure 4 shows, in a second step, how the closure strap 3 subsequently is wound for a second revolution around the body limb, partly overlapping the first revolution of the strap and partly in contact with the bare skin above the protective sleeve.

Through the above said application procedure is achieved, partly in cooperation with the inherent flexibility of the sleeve, which is an effect of the specific composition of polymers in the plastic, a close contact between the sleeve and the skin of the body part without channels forming in the pleats, partly in cooperation with the closure strap 3, a motion resistant securement of the sleeve pleating and also a securement of the closure area against the skin of the body limb.

The carrier of the adhesive layer of the closure strap 3, i.e. the tape 10, has a material inherent resilience, displaying a partially permanent deformation or linear expansion, which significantly reduces the risk of cutting off the blood flow in the wounded body limb. This refers to the limited elasticity of the strap, which provides a better control of the applied pressure, after finishing the manual stretching of the strap.

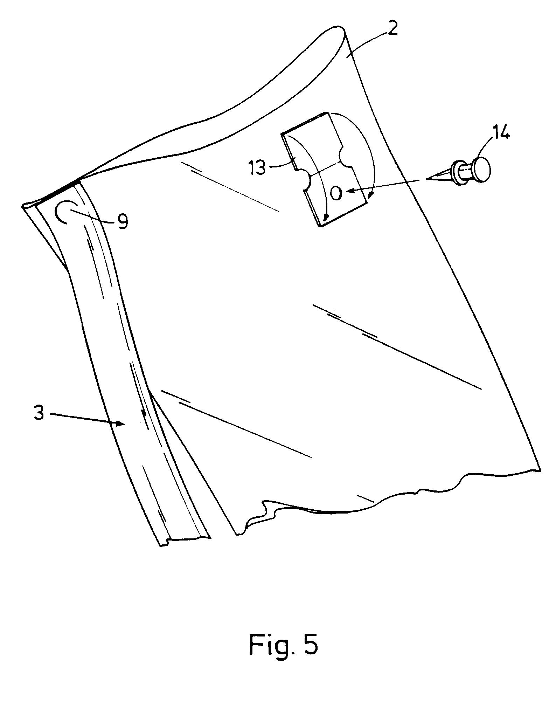

Figure 5 shows how the protective sleeve 2 can be supplementary provided with a ventilation valve 13 to improve the operation of the sleeve as a bath protection. When bathing, the water pressure tends to squeeze out the air that is enclosed in the sleeve, and which has to pass out by the closure area. Upon this escape of air, small channels are formed through which water can enter, when bathing during a certain period of time. To remove this problem, a ventilation valve is attached in conjunction with the application of the sleeve to the body part. The ventilation valve 13 comprises a foldable tab 13, provided on both sides with double sided tape. The tape is covered by a protective foil, which at least on one side can be divided at a folding line in the tab 13. In one end, the tab 13 has a centrally located hole, and is supplied together with a hole making tool 14.

The ventilation valve or tab 13 is by one half adhered to the outside of the sleeve 2, some to 10 cm spaced from the area that will be covered by the closure strap. The hole making tool 14 is plugged into the hole of the valve and penetrates the material of the protective sleeve 2, whereupon the later in above said manner is applied to the body limb. Subsequently, the body part is gradually lowered into the water, whereby the enclosed air escapes through the ventilation hole. Before the water has reached the ventilation valve 13, the protective foil is removed from the double sided tape, and the valve is folded over itself and thereby effectively sealing the ventilation hole. By removing, in this manner, a substantial part of the air that initially was enclosed in the protective sleeve, the utilization of the sleeve as a bath protection is significantly increased.

By forming, as in the present invention, a protective sleeve 2 from a thin, synthetic material tube with one single sealing line, and attaching a closure strap 3, running parallel with the tube, by a weld attachment 9 and cutting, to the desired length, both the sleeve and the strap, respectively, in one manufacturing step, is achieved a maximum simplified manufacturing process, whereby the production costs will be brought to the lowest level to meet the requirements for a disposable product. By orienting the closure strap parallel to the protective sleeve, it is likewise provided for low handling costs for package and storing.

The materials enclosed in the tape 10, adhesive layer 11 and protective foil 12 are already in practice within surgery and rehabilitation, where they have proved to be friendly to the skin and do not cause allergic reactions.

Above is described a shower and bath protection which in regard of details can be modified without diverging from the scope of invention as defined by the independant claim, hence, a granted protection should be interpreted also to include such modification which comes within the extent of the claim.

## Claims

1. Disposable protection for water tight enclosing of an extremity, including a protective sleeve (2) and a closure strap (3), said closure strap comprising a tape (10) which is provided with an adhesive layer (11) along its entire length, and with a protective foil (12) covering the adhesive layer (11), **characterized** by
- the protective sleeve (2) being produced by heat sealing and cutting to the desired length a tube shaped synthetic material,
- the closure strap (3) being attached to the sleeve (2), parallel to the longitudinal direction of the sleeve and preferably being of equal length to the protective sleeve (2), and by
- the closure strap (3) being attached to the protective sleeve (2) by a welding joint (9), connecting the closure strap with both layers of the double folded protective sleeve (2).

2. Disposable protection according to claim 1, **characterized** by the synthetic material in the protective sleeve (2) being polyethylene with an admixture of EBA (Ethylene-Butyl-Acrylate) to an amount in the range of 10-20 percentage by weight, preferably to an amount in the range of 15-18 percentage by weight.

3. Disposable protection according to claim 1, **characterized** by said tape (10), when stretched in the longitudinal direction, displaying a partially permanent linear expansion, and by said protective foil (12) being a silicone treated paper.

4. Disposable protection according to claim 1, **characterized** by a ventilation valve (13) optionally attachable to the outside of the protective sleeve (2).

5. Disposable protection according to claims 1 and 4, **characterized** by the ventilation valve (13) comprising a foldable tab (13), on both sides provided with an adhesive layer and a protective foil, covering the adhesive layers, which foil at least on its one side is divided along a folding line of the tab (13), and by the tab comprising a through hole at one end thereof.

6. Method for producing a disposable cover for water tight enclosing of an extremity according to claim 1, **characterized** by the steps of
- producing a sleeve by heat sealing (4) one end of a thin plastic tube,
- attaching a closure strap (3) to the sleeve, so that the closure strap is engaging and extending parallel to a folding edge of the flattened plastic tube, whereby the closure strap through a weld joint (9) is attached to both layers of the flattened plastic tube, and
- cutting the plastic tube, whereby coincidently the closure strap is cut to be of the same length as the plastic tube.

7. The utilization of polyethylene comprising an addition of EBA (Ethylene-Butyl-Acrylate) to an amount of 10 to 20 percentage by weight, preferably 15-18 percentage by weight, in manufacturing a disposable cover for water tightly enclosing an extremity.

## Patentansprüche

1. Einmalschutz zur wasserdichten Umhüllung einer Extremität, mit einer Hülle (2) und einem Verschlußstreifen (3), der ein Band (10) enthält, das über seine gesamte Länge mit einer Klebschicht (11) und einer die Klebschicht (11) bedeckenden Schutzfolie (12) versehen ist, **dadurch gekennzeichnet**,
- daß die Schutzhülle (2) hergestellt wird durch Heißsiegelung eines aus Synthetikmaterial geformten Schlauches und Zerschneiden auf die gewünschte Länge,
- daß der Verschlußstreifen (3) an der Hülle (2) parallel zu deren Längsrichtung und vorzugsweise mit gleicher Länge wie die Schutzhülle (2) befestigt wird, und
- daß der Verschlußstreifen (3) an der Schutzhülle (2) durch eine Schweißverbindung (9) angebracht wird, welche den Verschlußstreifen mit beiden Schichten der verdoppelt gefalteten Schutzhülle (2) verbindet.

2. Einmalschutz nach Anspruch 1, **dadurch gekennzeichnet**, daß das Synthetikmaterial in der Schutzhülle (2) Polyäthylen mit einer Zumischung von EBA (Äthylen-Butyl-Acrylat) bis zu einer Menge im Bereich von 10 bis 20 Gew.-%, vorzugsweise bis zu einer Menge im Bereich von 15 bis 18 Gew.-%, ist.

3. Einmalschutz nach Anspruch 1, **dadurch gekennzeichnet**, daß das Band (10) bei Streckung in Längsrichtung eine teilweise permanente lineare Dehnung zeigt, und daß die Schutzfolie (12) ein silikonbehandeltes Papier ist.

4. Einmalschutz nach Anspruch 1, **gekennzeichnet durch** ein wahlweise an der Außenseite der Schutzhülle (2) anbringbares Lüftungsventil (13).

5. Einmalschutz nach den Ansprüchen 1 und 4, **dadurch gekennzeichnet**, daß das Lüftungsventil (13) eine faltbare Lasche (13) aufweist, die auf beiden Seiten mit einer Klebschicht und einer die Klebschichten abdeckenden Schutzfolie versehen ist, welche mindestens an einer ihrer Seiten durch eine Faltlinie der Lasche (13) geteilt ist, und daß die Lasche an ihrem einen Ende ein Durchgangsloch aufweist.

6. Verfahren zur Herstellung einer Einmalhülle zur wasserdichten Umhüllung einer Extremität nach Anspruch 1, **gekennzeichnet durch** die Schritte:
- Herstellen einer Hülle durch Heißversiegeln (4) eines Endes eines dünnen Plastikschlauchs,
- Befestigen eines Verschlußstreifens (3) an der Hülle, so daß der Verschlußstreifen an einer Faltkante des abgeflachten Plastikschlauches angreift und parallel zu ihr verläuft und mit beiden Schichten des abgeflachten Plastikschlauchs durch eine Schweißverbindung (9) verbunden ist, und
- Zerschneiden des Plastikschlauchs, wobei gleichzeitig der Verschlußstreifen so geschnitten wird, daß er dieselbe Länge wie der Plastikschlauch hat.

7. Verwendung von Polyäthylen mit einem Zusatz von EBA (Äthylen-Butyl-Acrylat) in einer Menge von 10 bis 20 Gew.-%, vorzugsweise 15 bis 18 Gew.-%, zur Herstellung einer Einmalhülle zur wasserdichten Umhüllung einer Extremität.

## Revendications

1. Protection jetable pour entourer de façon étanche à l'eau une extrémité, comprenant un manchon protecteur (2) et une bande de fermeture (3), ladite bande de fermeture comportant un ruban (10) qui est pourvu d'une couche adhésive (11) sur toute sa longueur et d'une pellicule protectrice (12) couvrant la couche adhésive (11), caractérisée en ce que :
le manchon protecteur (2) est produit par thermosoudage et coupe à la longueur désirée d'une matière synthétique en forme de tube,
la bande de fermeture (3) est attachée au manchon (2), parallèlement à la direction longitudinale du manchon, et elle est de préférence de même longueur que le manchon protecteur (2), et
la bande de fermeture (3) est attachée au manchon protecteur (2) par une jonction soudée (9), reliant la bande de fermeture aux deux couches du manchon protecteur plié à plat (2).

2. Protection jetable suivant la revendication 1, caractérisée en ce que la matière synthétique du manchon protecteur (2) est du polyéthylène avec mélange de EBA (Ethylène-Butyl-Acrylate) en une quantité comprise entre 10 et 20% en poids,et de préférence en une quantité comprise entre 15 et 18% en poids.

3. Protection jetable suivant la revendication 1, caractérisée en ce que ledit ruban (10), lorsqu'il est tendu dans la direction longitudinale, présente un allongement linéaire partiellement permanent, et en ce que ladite pellicule protectrice (12) est un papier siliconé.

4. Protection jetable suivant la revendication 1, caractérisée en ce qu'elle comprend une valve de ventilation (13) qui peut être fixée optionnellement à l'extérieur du manchon protecteur (2).

5. Protection jetable suivant les revendications 1 et 4, caractérisée en ce que la valve de ventilation (13) comprend une patte pliable (13) pourvue sur ses deux faces d'une couche adhésive et d'une pellicule protectrice qui couvre les couches adhésives, la dite pellicule étant divisée au moins sur sa première face le long d'une ligne de pliage de la patte (13), et en ce que la patte comporte un trou traversant à une de ses extrémités.

6. Procédé de fabrication d'une protection jetable pour entourer de façon étanche à l'eau une extrémité suivant la revendication 1, caractérisé par les étapes de :
production d'un manchon par thermosoudage (4) d'une extrémité d'un tube en matière plastique mince,
attache d'une bande de fermeture (3) au manchon, de sorte que la bande de fermeture est en contact avec un bord de pliage du tube en matière plastique aplati et est sensiblement parallèle à ce bord, la bande de fermeture étant attachée par une jonction soudée (9) aux deux couches du tube en matière plastique aplati, et
coupe du tube en matière plastique, de sorte que la bande de fermeture est coupée en coÏncidence à la même longueur que le tube en matière plastique.

7. Utilisation de polyéthylène comprenant une addition de EBA (Ethylène-Butyl-Acrylate) à raison de 10 à 20% en poids, et de préférence 15 à 18% en poids, dans la fabrication d'une protection jetable pour entourer de façon étanche à l'eau une extrémité.
